# EUROPEAN PATENT APPLICATION

(11) **EP 4 130 279 A1**
(43) Date of publication of application: **08.02.2023**
(21) Application number: 21781096.9
(22) Date of filing: 29.01.2021
(51) Int. Cl.: C12N 15/62, C12N 15/70, C07K 19/00, C07K 14/00, C07K 16/00, C07K 1/22

(54) **FUSION PROTEIN OF Z-DOMAIN AND CALSEQUESTRIN, HAVING IMPROVED REACTIVITY, STABILITY, AND ANTIBODY RECOVERY, AND METHOD FOR ISOLATION AND PURIFICATION OF ANTIBODY USING SAME**

(30) Priority: 30.03.2020 KR 20200038530
(71) Applicant: Korea Institute of Ceramic Engineering and Technology, Jinju-si, Gyeongsangnam-do 52851 (KR)
(72) Inventor: KIM, Sung Hyun, Sejong-si 30098 (KR); PARK, Hee Sun, Cheongju-si, Chungcheongbuk-do 28171 (KR)
(74) Representative: Appleyard Lees IP LLP
(86) International application number: PCT/KR2021/001233
(87) International publication number: WO 2021/201405

(57) **Abstract**

The present disclosure relates to a fusion protein of Z-domain and calsequestrin having improved reactivity, stability, and antibody recovery, and a method of isolating and purifying antibodies using the same. Specifically, the present disclosure relates to: a nucleic acid encoding a fusion protein of Z-domain and calsequestrin having improved reactivity, stability, and antibody recovery; a recombinant expression vector including the nucleic acid; a host cell transformed with the recombinant expression vector; and a method of isolating and purifying antibodies by using the fusion protein of Z-domain and calsequestrin having improved reactivity, stability, antibody recovery, and purity.

## Description

### TECHNICAL FIELD

The present disclosure relates to a Z-domain- and calsequestrin fusion protein having improved reactivity, stability, and antibody recovery, and a method of isolating and purifying antibodies using the same. Specifically, the present disclosure relates to: a nucleic acid encoding a Z-domain-calsequestrin fusion protein having improved reactivity, stability, and antibody recovery; a recombinant expression vector including the nucleic acid; a host cell transformed with the recombinant expression vector; and a method of isolating and purifying antibodies using the Z-domain-calsequestrin protein having improved reactivity, stability, antibody recovery, and purity.

### BACKGROUND ART

A method for purifying monoclonal antibodies typically includes four basic steps. These steps are (1) Harvest -separation of host cells from the fermentation culture; (2) Capture - separation of antibodies from the majority of components in the harvested culture; (3) Micropurification - removal of residual host cell debris and aggregates; and (4) Formulation - preparation of the antibodies in a suitable carrier for maximum stability and shelf life. However, these steps do not necessarily result in generation of antibodies with acceptable purity for use in a pharmaceutical setting. Therefore, it is important to have a method of generating and purifying antibodies of interest in a pure form from which impurities are removed to the extent suitable for a pharmaceutical use.

Protein A chromatography has been widely used in industrial production of antibodies because it enables almost complete purification of antibodies, usually IgGs, from cell culture supernatant in a single step. However, a protein A chromatography column has an issue in that repetitive use of the column result in some loss of ligands therefrom. Such protein A or protein A fragment has affinity for IgG, forms a complex with the antibody and contaminates the antibody, and is also difficult to remove from the purified antibody. In particular, since protein A, a bacterial protein may induce an undesired immune response, the protein needs to be removed from tpurified antibodies. Therefore, the antibody purification process using the protein A chromatography has a drawback in that there is a need to monitor and remove a residual amount of protein A for each process.

In this regard, the present inventors disclosed a method of purifying antibodies with high purity and high quality without using expensive protein A chromatography commonly used for antibody purification in Korean Patent No. 10-1774354 (Title of the disclosure: Method of isolating and purifying antibodies using Z-domain-calsequestrin fusion protein).

### Prior Art Document

### 1. Korean Patent No. 10-1774354

### DESCRIPTION OF EMBODIMENTS

### TECHNICAL PROBLEM

The present inventors have continued research on isolation and purification of antibodies to find that the reactivity with calcium, stability, antibody recovery, and purity of a Z-domain-calsequestrin fusion protein may be significantly increased by changing the amino acid sequence of the Z-domain consisting of an amino acid sequence of SEQ ID NO: 1 and calsequestrin consisting of an amino acid sequence of SEQ ID NO: 4 in the Z-domain-calsequestrin fusion protein disclosed in Patent No. 10-1774354 may lead to significant increase in reactivity with calcium and stability thereof and recovery yield and purity of antibodies obtained by use thereof, .

### SOLUTION TO PROBLEM

The present disclosure relates to a nucleic acid encoding a Z-domain - calsequestrin fusion protein with improved reactivity, stability, and antibody recovery, wherein the calsequestrin consists of an amino acid sequence of SEQ ID NO: 5 or SEQ ID NO: 6.

The present disclosure also relates to a recombinant expression vector including the nucleic acid encoding the Z-domain-calsequestrin with improved reactivity, stability, and antibody recovery.

The present disclosure also relates to a host cell transformed with the recombinant expression vector including the nucleic acid encoding the Z-domain-calsequestrin fusion protein with improved reactivity, stability, and antibody recovery.

The present disclosure also relates to a method of isolating and purifying antibodies with improved reactivity, stability, and antibody recovery, the method comprising:
A) preparing a recombinant vector including a nucleic acid encoding a Z-domain (Z)-calsequestrin (CSQ) fusion protein;
B) transducing the recombinant vector into a host cell to obtain a transformant;
C) expressing the Z-CSQ fusion protein in the transformant; and
D) isolating antibodies bound to the Z-CSQ fusion protein from the transformant by using calcium,
wherein the calsequestrin consists of an amino acid sequence of SEQ ID NO: 5 or SEQ ID NO: 6.

### ADVANTAGEOUS EFFECTS OF DISCLOSURE

A fusion protein of Z-domain and calsequestrin according to the present disclosure exhibits higher stability than the fusion protein disclosed in Korean Patent No. 10-1774354, and improved precipitation reaction with calcium and antibody recovery rate, and thus can be used for high-throuput isolation and purification of antibodies compared to the protein A chromatography column widely used in the industry, thereby reducing purification costs and preventing antibody degradation. In addition, the fusion protein of Z-domain and calsequestrin may be regenerated for reuse by using a chelate, and is thus, economical.

### BRIEF DESCRIPTION OF DRAWINGS

FIG. 1 shows results of an experiment on turbidity of ZZ-CSQ1 (Full) and ZZ-CSQ2 (Δ) by calcium concentration, according to Experimental Example 1.
FIG. 2 shows results of an experiment confirming the stability of ZZ-CSQ1 (Full) and ZZ-CSQ2 (Δ), according to Experimental Example 1.
FIG. 3 shows results of an experiment confirming stability of ZZ-CSQ1 (Full) according to Experimental Example 1 after G4S, a linker of ZZ-CSQ1 (Full), is replaced by 4N, 8N, and LE8N, respectively.
FIG. 4 shows results of an experiment measuring affinity of fusion protein ZZ-CSQ1 (Full) to an antibody, according to Experimental Example 1.
FIG. 5 shows results of an experiment confirming purification of antibodies from an antibody culture medium by using ZZ-CSQ1 (Full) fusion protein, according to Experimental Example 1.
FIG. 6 shows results of an experiment confirming antibody recovery by ZZ-CSQ1 (Full) depending on pH, according to Experimental Example 1.
FIG. 7 shows results of an experiment identifying host cell protein (HCP) of antibodies after purification of the antibodies by using the fusion protein ZZ-CSQ1 (Full), according to Experimental Example 1.
FIG. 8 shows results of an experiment identifying host cell DNA (HCD) of antibodies after purification of the antibodies by using the fusion protein ZZ-CSQ1 (Full), according to Experimental Example 1.
FIG. 9 shows results of SEC-HPLC (size-exclusion high-performance liquid chromatography) showing aggregation of antibodies or ZZ-CSQ1 (Full) after purification of the antibodies by using the fusion protein ZZ-CSQ1 (Full), according to Experimental Example 1.
FIG. 10 shows results of an experiment measuring antibody recovery after changing a ZZ-domain in the fusion protein to Z6G and ZL4G, respectively, according to Experimental Example 1.
FIG. 11 shows results of an experiment on turbidity of ZZ-CSQ2 (Full) and ZZ-CSQ2 (Δ) by calcium concentration, according to Experimental Example 2.
FIG. 12 shows results of an experiment confirming antibody recovery of ZZ-CSQ2 (Full) and ZZ-CSQ2 (Δ) according to Experimental Example 2.
FIG. 13 shows results of an experiment confirming changes in stability after G4S, a linker of ZZ-CSQ1 (Full), is replaced by 4N, 8N, and LE8N, respectively, according to Experiment 2.
FIG. 14 shows results of an experiment confirming that only antibodies were isolated by using fusion protein ZZ-CSQ2 (Full) in an antibody culture medium, according to Experimental Example 2.
FIG. 15 shows results of an experiment measuring affinity of fusion protein Z-CSQ1 (Full) with an antibody, according to Experimental Example 3.
FIG. 16 shows results of an experiment measuring affinity of fusion protein (Z)₅-CSQ1 (Full) to an antibody, according to Experimental Example 4.
FIG. 17 shows results of an experiment confirming a binding ratio of (Z)₅-CSQ1 (Full) and antibodies, according to Experimental Example 5.

According to the first embodiment, the present disclosure is to provide a nucleic acid encoding a fusion protein of Z-domain and calsequestrin with improved reactivity, stability, and antibody recovery.

The term "fusion protein" as used herein refers to a protein prepared by joining two or more genes, each of which encodes a respective protein. The fusion protein may be artificially prepared by using recombinant DNA technology. In addition, the fusion protein may be in a form in which a bioactive protein or peptide and an immunoglobulin Fc fragment are linked without a linker or via a peptide linker. Here, a peptide linker refers to a peptide that connects two different proteins constituting a fusion protein. The peptide linker may be a linker included in such a way that the two proteins which are components of a fusion protein are respectively folded even in a form of the fusion protein or maintain their respective functions, but is not limited thereto.

The term "immunoglobulin Fc fragment" as used herein refers to the heavy chain constant region 2 (CH2) and the heavy chain constant region 3 (CH3) portion, excluding the variable regions of the heavy and light chains and the heavy chain constant region 1 (CH1) and the light chain constant region 1 (CL1), and may include a hinge portion in the heavy chain constant regions. Also, as long asthe immunoglobulin Fc fragment of the present disclosure has a substantially equivalent or enhanced effect compared to the native Fc, the immunoglobulin Fc fragment may be an extended Fc fragment including a part or all of heavy chain constant region 1 (CH1) and/or light chain constant region 1 (CL1) with exclusion of heavy and light chain variable regions of immunoglobulin only. The immunoglobulin Fc fragment may also be a region in which some fairly long amino acid sequences corresponding to CH₂ and/or CH₃ are removed. The immunoglobulin Fc fragment of the present disclosure not only includes native amino acid sequence but also sequence derivatives thereof. An amino acid sequence derivative refers to one having at least one amino acid residue different from the native amino acid sequence, and may occur naturally or artificially. Fc fragments of immunoglobulins include derivatives derived by deletion, insertion, non-conservative or conservative substitution, or a combination thereof. Insertions are usually of a continuous sequence of about 1 to 20 amino acids, although larger insertions are possible. Deletions are usually of about 1 to 30 residues. Techniques for preparing a sequence derivative of such an immunoglobulin Fc fragment is disclosed in International Publication No. WO97/34631 and International Publication No. WO96/32478, which are incorporated herein by reference. Amino acid exchanges in proteins and peptides that do not entirely alter the activity of the molecule are known in the art (H. Neurath, R.L. Hill, The Proteins, Academic Press, New York, 1979). The most common exchanges are exchanges between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, the immunoglobulin Fc fragment may be modified by phosphorylation, sulfation, acrylation, glycosylation, methylation, farnesylation, acetylation, amidation, or the like. Such immunoglobulin Fc fragments may be obtained from the natural-type isolated from animals such as humans, cows, goats, pigs, mice, rabbits, hamsters, rats, and guinea pigs, or may be a recombinant-type or a derivative thereof obtained from transfected animal cells or transformed microbial cells. Here, a method of obtaining immunoglobulin Fc fragment from the natural-type may be by isolating whole immunoglobulins from a living body of a human or animal, and then treating the same with a proteolytic enzyme. When treated with papain, the immunoglobulin is cleaved into Fab and Fc, and when treated with pepsin, the immunoglobulin is cleaved into pF'c and F(ab')2. Fc or pF'c may be separated by using size-exclusion chromatography or the like. Preferably, the immunoglobulin Fc fragment according to the present disclosure may include Protein A, Protein G, Protein A/G or Protein L derived from a microorganism.

The term "Z protein", used herein, refers to a protein obtained from Protein A of *Staphylococcus aureus* and used for defense against antibodies present in host cells. The Z protein is used in a self-defense mechanism by binding to the Fc region of an antibody in a host cell to prevent phagocytosis by macrophages. A tandem repeat dimer obtained by manipulating only the antibody-binding site in Protein A is referred to as a "Z-domain".

The term "calsequestrin", used herein, refers to a calcium-binding protein of the sarcoplasmic reticulum, which binds to calcium after muscle contraction even when the calcium concentration in the sarcoplasmic reticulum is higher than that in the cytoplasm to allow storage of calcium ions in the cisterna of the sarcoplasmic reticulum. Calsequestrin has a high calcium storage capacity, because one calsequestrin molecule can bind to multiple calcium ions (for example, each calsequestrin molecule has 40 to 50 calcium binding sites).

In the nucleic acid according to the present disclosure, the Z-domain is in a form of Zn (n is an integer equal to or higher than 1) in which Z-domains are repeated. For example, the Z-domain may be in a form in which one or more, or 1 to 20, or 1 to 10, or 1 to 5 Z-domains are repeated. Preferably, the Z-domain may be in a form in which two to five Z-domains are repeated.

In the nucleic acid according to the present disclosure, the Z-domain consists of an amino acid sequence of SEQ ID NO: 13 and has a form having 1 to 5 repeats.

In the nucleic acid according to the present disclosure, the Z-domain is a ZZ-domain, a Z6G domain or a ZL4G domain. The ZZ-domain may include or consist of an amino acid sequence of SEQ ID NO: 1. The Z6G domain may include or consist of an amino acid sequence of SEQ ID NO: 2. The ZL4G domain may include or consist of an amino acid sequence of SEQ ID NO: 3.

In the nucleic acid according to the present disclosure, the calsequestrin may include or consist of an amino acid sequence of SEQ ID NO: 5 or SEQ ID NO: 6.

In the nucleic acid according to the present disclosure, the Z-domain and the calsequestrin are fused via a linker, which includes or consists of one or more amino acid sequences selected from the group consisting of SEQ ID NOs: 7 to 10.

In the nucleic acid according to the present disclosure, the antibody is an antibody, or a fragment, derivative, or analog htereof, including an Fc capable of specifically binding to the Z protein, a fragment thereof, or derivative/analog thereof. For example, the antibody may be IgG.

According to the second embodiment,
provided is a method of isolating and purifying antibodies with improved reactivity and stability, including: (A) preparing a recombinant vector including a nucleic acid encoding a Z-domain- calsequestrin fusion protein;
(B) transferring the recombinant vector into a host cell to obtain a transformant;
(C) expressing the Z-calsequestrin protein in the transformant; and
(D) isolating antibodies bound to the Z-domain-calsequestrin fusion protein from the expressed transformant by using calcium.

The term "vector", used herein, refers to an expression vector capable of expressing a protein of interest in a suitable host cell, and refers to a nucleic acid construct including essential regulatory elements operably linked to express a nucleic acid insert. Such a vector may be one prepared by manipulating plasmids (for example, pSC101, ColE1, pBR322, pUC8/9, pHC79, pGEX series, pET series, pACYC184, pUC19, etc.), phages (for example, λ-gt4, λB , λ-Charon, λΔz1, M13, etc.) or viruses (for example, SV40, etc.) often used in the art, but is not limited thereto.

The term "transformation", used herein, refers to introducing DNA into a host so that the DNA may be replicated as a extrachromosmal element or by integration into chromosome, and means a phenomenon of artificially making a genetic change by introducing external DNA into a cell. The transformation method includes CaCl₂ precipitation method, Hanahan method with increased efficiency by using dimethyl sulfoxide (DMSO) as a reducing material in the CaCl₂ method, electroporation, calcium phosphate precipitation method, protoplast fusion method, agitation method using silicon carbide fiber, agrobacterium-mediated transformation method, transformation method using PEG (polyethylene glycol), dextran sulfate, lipofectamine and drying/inhibition-mediated transformation method, but is not limited thereto.

The term "host cell", used herein, refers to a cell that supplies nutrients to other parasitic microorganisms or genes, and is a cell, when transformed by a vector, to which various genetic or molecular influences are exerted by the vector. The host cell may include *Escherichia* sp. bacteria such as *E. coli*; *Bacillus* sp. such as *Bacillus subtili*; *Pseudomonas* sp. such as *Pseudomonas putida*; lactic acid bacteria such as *Lactobacillus* and *Enterococcus*; yeasts such as *Saccharomyces cerevisiae*, or *Schizosaccharomyces pombe*; animal cells or insect cells, but is not limited thereto.

In the method of isolating and purifying antibodies according to the present disclosure, the Z-domain includes Zn (n is an integer equal to or higher than 1), which is a form in which the Z-domain is repeated. For example, the Z-domain may be in a form in which one or more, 1 to 20, 1 to 10, or 1 to 5 Z-domains are repeated. Preferably, the Z-domain may be in a form in which two to five Z-domains are repeated.

In the nucleic acid according to the present disclosure, the Z-domain consists of an amino acid sequence of SEQ ID NO: 13 and has a form in which 1 to 5 Z-domains are repeated.

In the method of isolating and purifying antibodies according to the present disclosure, the Z-domain is a ZZ-domain, a Z6G domain or a ZL4G domain. The ZZ-domain may include or consists of an amino acid sequence of SEQ ID NO: 1. The Z6G domain may include or consists of an amino acid sequence of SEQ ID NO: 2. The ZL4G domain may include or consists of an amino acid sequence of SEQ ID NO: 3.

In the method of isolating and purifying antibodies according to the present disclosure, the calsequestrin includes or consists of an amino acid sequence of SEQ ID NO: 5 or SEQ ID NO: 6.

In the method of isolating and purifying antibodies according to the present disclosure, the Z-domain and the calsequestrin are fused via a linker which includes or consists of one or more amino acid sequences selected from the group consisting of SEQ ID NOs: 7 to 10.

In the method of isolating and purifying antibodies according to the present disclosure, the antibody is an antibody, or a fragment, derivative, or an analog thereof, including an Fc capable of specifically binding to the ZZ protein, a fragment thereof, or a derivative/analog thereof. For example, the antibody may be IgG.

In the method of isolating and purifying antibodies according to the present disclosure, the step (D) is conducted under the conditions of pH 3.0 to pH 4.5. Preferably, the step (D) may be carried out under the conditions of pH 3.0 to pH 3.5.

In the method of isolating and purifying antibodies according to the present disclosure, the step (D) of isolating antibodies bound to the Z-domain-calsequestrin fusion protein by using calcium includes:
(d-1) precipitating the antibodies bound to the Z-domain-calsequestrin fusion protein by using calcium; and
(d-2) removing the precipitate through centrifugation or filtration.

### MODE OF DISCLOSURE

Unless defined otherwise herein, all technical and scientific terms used have the same meaning as commonly understood by one of ordinary skill in the art, and the present disclosure will be described in more detail by the following Examples with reference to the accompanying drawings. However, these Examples are only for an illustrative purpose, and the scope of the present disclosure is not limited thereto.

### <Examples>

### Example 1. Preparation of Z-domain-calsequestrin fusion protein

Z-domain-calsequestrin fusion proteins including a Z-domain, a linker, and calsequestrin, each of which consists of the amino acid sequence shown in Table 1 below were designed. On the other hand, as a comparative example, the fusion protein of Korean Patent No. 10-1774354 was used.

**[Table 1]**

| No. | Amino acid SEQ ID NO | | | |
|---|---|---|---|---|
| | Fusion protein | Z-domain | Linker SEQ | Calsequestrin |
| Comparative Example | ZZ-CSQ2(Δ) | SEQ ID NO: 1 | SEQ ID NO: 7 | SEQ ID NO: 4 |
| Preparation Example 1 | ZZ-CSQ1 (Full) | SEQ ID NO: 1 | SEQ ID NO: 7 | SEQ ID NO: 5 |
| Preparation Example 2 | ZZ-CSQ1 (Full) | SEQ ID NO: 1 | SEQ ID NO: 8 | SEQ ID NO: 5 |
| Preparation Example 3 | ZZ-CSQ1 (Full) | SEQ ID NO: 1 | SEQ ID NO: 9 | SEQ ID NO: 5 |
| Preparation Example 4 | ZZ-CSQ1 (Full) | SEQ ID NO: 1 | SEQ ID NO: 10 | SEQ ID NO: 5 |
| Preparation Example 5 | ZZ-CSQ1 (Full) | SEQ ID NO: 2 | SEQ ID NO: 7 | SEQ ID NO: 5 |
| Preparation Example 6 | ZZ-CSQ1 (Full) | SEQ ID NO: 3 | SEQ ID NO: 7 | SEQ ID NO: 5 |
| Preparation Example 7 | ZZ-CSQ1 (Full) | SEQ ID NO: 1 | SEQ ID NO: 7 | SEQ ID NO: 6 |
| Preparation Example 8 | ZZ-CSQ1 (Full) | SEQ ID NO: 1 | SEQ ID NO: 8 | SEQ ID NO: 6 |
| Preparation Example 9 | ZZ-CSQ1 (Full) | SEQ ID NO: 1 | SEQ ID NO: 9 | SEQ ID NO: 6 |
| Preparation Example 10 | ZZ-CSQ1 (Full) | SEQ ID NO: 1 | SEQ ID NO: 10 | SEQ ID NO: 6 |
| Preparation Example 11 | ZZ-CSQ1 (Full) | SEQ ID NO: 13 | SEQ ID NO: 7 | SEQ ID NO: 5 |
| Preparation Example 12 | ZZ-CSQ1 (Full) | SEQ ID NO: 14 | SEQ ID NO: 7 | SEQ ID NO: 5 |

### Example 2. Cloning of gene for Z-domain-calsequestrin fusion protein

The nucleic acid encoding the Z-domain-calsequestrin fusion protein was amplified by using PCR (polymerase chain reaction) to clone the same into the expression vector pET28b. To this end, a forward primer (AATAAAA CATATG GTAGACAACAAATTCAAC, SEQ ID NO: 11) and a reverse primer (ATT CTCGAG TTA ATCATCATCATCATCATCTTC, SEQ ID NO: 12) were designed (Bioneer). The PCR product was purified by using a DNA purification kit (GeneAll) and treated with Ndel and Xhol restriction enzymes (restriction site sequences indicated by the underlined region in the primers as above, NEB). In addition, the pET28b vector (Novagen) was also treated with Ndel and Xhol restriction enzymes. The DNA fragment and the pET28b vector digested with Ndel and Xhol were ligated by using a ligase (NEB) at 16 °C for 12 hours. The ligated product was transformed into DH5a *E. coli* and cultured for one day in an agar medium containing Kanamycin, and then the plasmid was extracted from the grown colony and sequenced (Bioneer).

### Example 3. Expression and purification of Z-domain-calsequestrin fusion protein

*Escherichia coli* strain BL21(DE3) (Novagen, Northumberland, UK) was transformed with the plasmid prepared in Example 2. The bacteria were treated with 1 mM isopropyl β-D-thiogalactopyranoside (IPTG) (Sigma, St. Louis, MO) at 37 °C for 6 hours to induce expression of the transformed gene. Afterwards, the bacteria were harvested and resuspended in a lysis buffer (50 mM sodium phosphate, pH 8.0, 300 mM NaCl and 5 mM imidazole) and lysed by sonication. The lysate was centrifuged at 1,550 g at 4 °C for 1 hour. The supernatant was loaded onto a gravity-flow columm (BioRad, Hercules, CA) packed with Ni-NTA affinity resin (Peptron, Daejeon, Korea) pre-equilibrated with a lysis buffer (bed capacity of 3 mL per liter of culture medium). After washing the column and matrix with a lysis buffer in an amount 10 times the bead volume, calsequestrin was eluted and obtained. The protein was further purified by gel filtration using a Superdex 200 column (Amersham PHarmacia, Bucks, UK) pre-equilibrated with a phosphate buffer solution (PBS, pH 7.4).

### <Experimental Examples>

### Experimental Example 1: Confirmation of reactivity and stability of fusion protein including calsequestrin (CSQ) consisting of amino acid sequence of SEQ ID NO: 5

### 1-1. Comparison of ZZ-CSQ1 and ZZ-CSQ2 (Δ)

### (1) Calcium reactivity

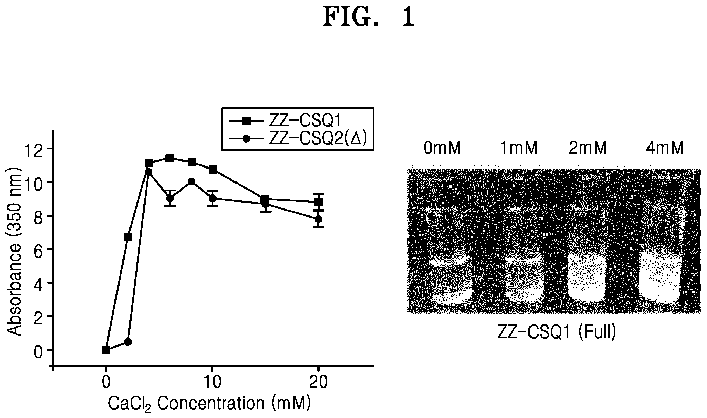

In order to examine the turbidity of the ZZ-CSQ1 (full) fusion protein according to Preparation Example 1 and the ZZ-CSQ2 (Δ) fusion protein according to Comparative Example, each protein was made to a concentration of 1 mg/ml, and the turbidity was measured. As a buffer for the fusion protein, 20 mM Tris-HCl (pH 7.0) was used, and calcium was added to the fusion protein at concentrations of 0, 2, 4, 6, 8, 10, and 20 mM, mixed, and incubated at 4 °C for 1 hour so that the fusion proteins react with calcium. Absorbance values for each concentration were measured at UV-Vis (350 nm) by using Nano-drop (Thermo). As a result, ZZ-CSQ1 (Full) and ZZ-CSQ2 (Δ) were found to react with calcium at a concentration of 2 mM and 4 mM, respectively, showing that ZZ-CSQ1 (Full) according to Preparation Example 1 reacts with calcium and precipitate at a lower concentration than ZZ-CSQ2 (Δ) (FIG. 1).

### (2) Stability

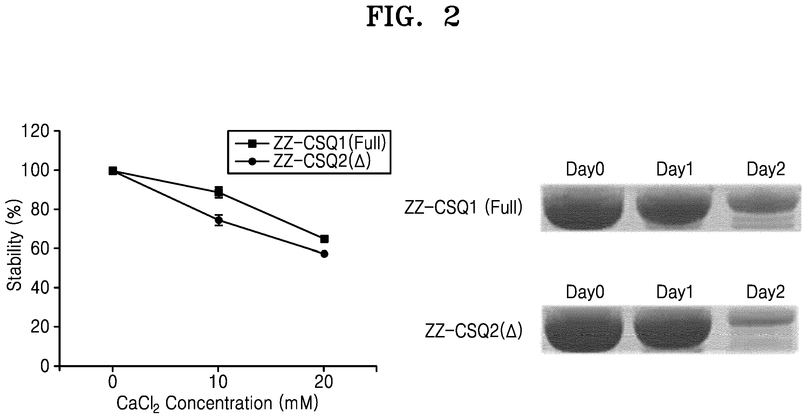

In order to examine the stability of ZZ-CSQ1 (Full) and ZZ-CSQ2 (Δ) fusion proteins, the fusion proteins ZZ-CSQ1 (Full) and ZZ-CSQ2 (Δ) at a concentration of 1 mg/ml were placedin a 37 °C incubator for 1 day and 2 days. In order to find any change in stability of the fusion protein over time, samples were taken at the indicated times, 4X sample buffer was added to each sample, then, the sample was heat-treated at 80 °C for 10 minutes followed by polyacrylamide gel electrophoresis (SDS-PAGE) for identification. As a result, it was found that the ZZ-CSQ2 (Δ) degraded more over time than ZZ-CSQ1 (Full) (FIG. 2).

### (3) Stability by type of linker

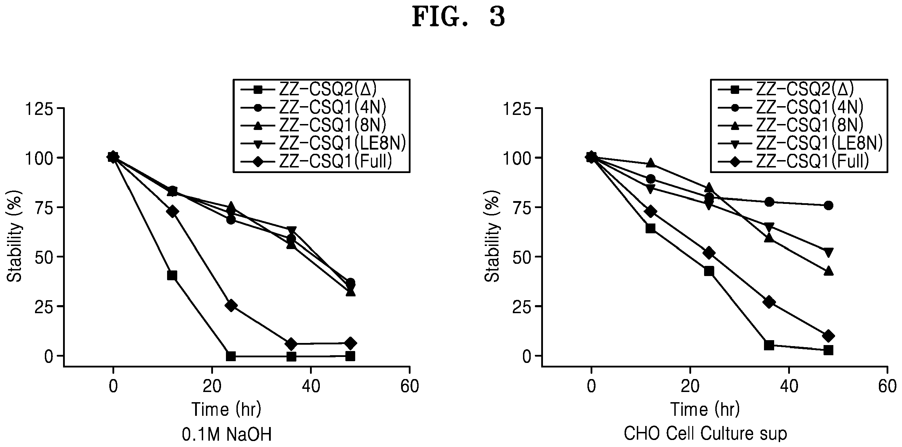

After changing G4S (SEQ ID NO: 7), which is a linker part of the ZZ-CSQ1 (Full) fusion protein, to 4N (SEQ ID NO: 8), 8N (SEQ ID NO: 9) and LE8N (SEQ ID NO: 10), respectively, 1 mg/ml of each of the fusion proteins ZZ-CSQ1 (Full) and ZZ-CSQ2 (Δ) according to Preparation Examples 1 to 4 was treated with 0.1 M of NaOH and CHO Cell Culture Sup. (supernatant obtained from CHO cell culture) in an incubator at 25 °C or 37 °C for 12, 24, 36, or 48 hours, in order to examine stability. In order to find any change in stability of the fusion protein over time, samples were taken at the indicated times, 4X sample buffer was added to each sample, then, the sample was heat-treated at 80 °C for 10 minutes followed by polyacrylamide gel electrophoresis (SDS-PAGE) for identification. As a result, the ZZ-CSQ2 (Δ) fusion protein was rapidly or completely degraded after 24 hours, whereas each of ZZ-CSQ1 (Full) fusion proteins according to Preparation Examples 2 to 4, in which the linker was changed to 4N, 8N and LE8N, respectively, showed a stability of 50 % or more in CHO Cell Culture Sup, and 70 % or more in 0.1 M NaOH in 24 hours, confirming the increase in the reuse rate (FIG. 3).

### 1-2. Measurement of affinity of ZZ-CSQ1 (Full) fusion protein to antibody

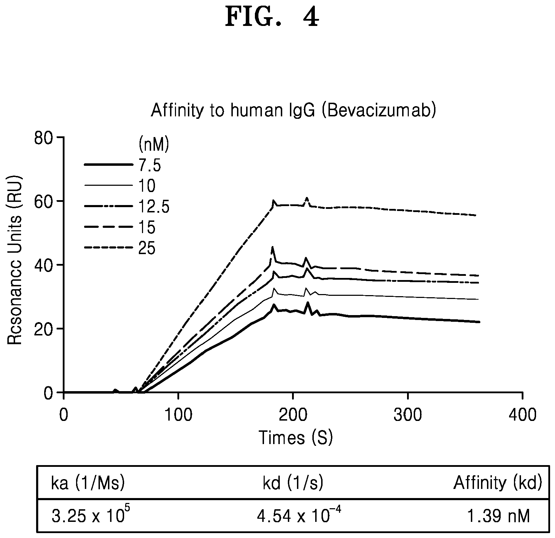

Surface plasmon resonance (SPR) analysis was performed to measure the affinity of the ZZ-CSQ1 (Full) fusion protein according to Preparation Example 1. ZZ-CSQ1 (Full) was immobilized on a CM5 chip, and Bevacizumab in a buffer solution of 50 mM Tris at pH 7.4 in various concentrations (7.5, 10, 12.5, 15 and 25 nM) was injected at a flow rate of 30 µl/min. Kinetics data were analyzed with BIA evaluation 2.1 software (GE Healthcare). As a result, the divalent Z-domain of ZZ-CSQ1 (Full) according to Preparation Example 1 was found to be capable of binding strongly to an antibody with an affinity of 1.39 nM (FIG. 4).

### 1-3. Purification of antibody by using ZZ-CSQ1 (Full) fusion protein

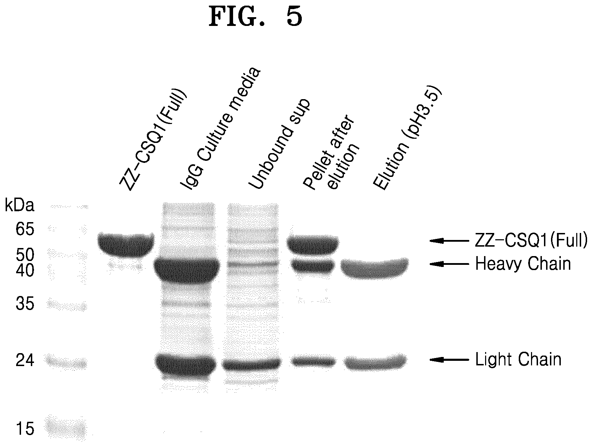

A process of purifying only antibodies by using the ZZ-CSQ1 (Full) fusion protein according to Preparation Example 1 from a culture of cells expressing antibodies (antibody culture medium) was examined. ZZ-CSQ1 (Full) fusion protein was added to the antibody culture medium at a ratio of 1:1, and reacted at 4 °C for 30 minutes. 10 mM calcium was added to the culture medium and reacted at 4 °C for 30 minutes followed by centrifugation at 4,000 g for 30 minutes to separate it into a supernatant and pellets. In order to elute antibodies from ZZ-CSQ1(Full)+antibody, 50 mM citrate (pH 3.5) was used. In order to confirm antibody recovery level, 4X sample buffer was added to the eluate, the resulting mixture was heat-treated at 80 °C for 10 minutes, and then identified by polyacrylamide gel electrophoresis (SDS-PAGE). As a result, only antibodies (IgG) were found eluted from the ZZ-CSQ1 (Full) according to Preparation Example 1+antibody (IgG)(FIG. 5).

### 1-4. Antibody recovery by ZZ-CSQ1 (Full) fusion protein depending on pH

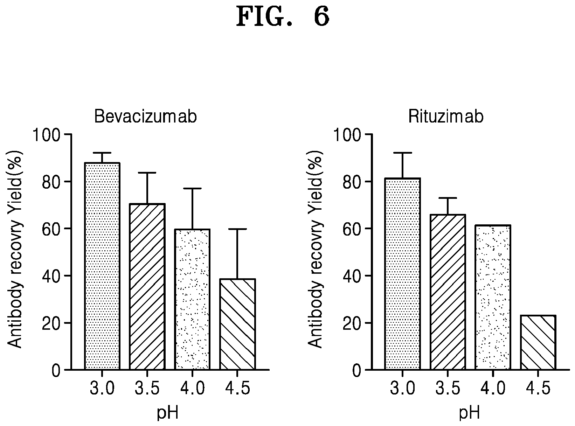

In order to identify antibody recovery yield depending on pH, ZZ-CSQ1 (Full) according to Preparation Example 1 was added to antibodies at a ratio of 1:1, and incubated at 4 °C for 30 minutes followed by centrifugation at 180,000 g for 30 minutes to separate the mixture into a supernatant and pellets. 100 mM of citrate buffer (pH 3.0, 3.5, 4.0, and 4.5) was added to the ZZ-CSQ1 (Full)+antibody (IgG) complex to elute the antibodies. After the elution, the eluate was centrifuged at 4,000 g for 30 minutes to separate a supernatant from pellets, and in order to identify the elution level of the antibody, 4X Sample Buffer was added to the supernatant, the mixture was heat-treated at 80 °C for 10 minutes followed by polyacrylamide gel electrophoresis (SDS-PAGE) for identification. As a result, when the pH of the recovery buffer was more acidic (pH 3.0 and pH 3.5), antibody elution was found to be higher than that when the pH was 4.0 and 4.5 (FIG. 6).

### 1-5. Measurement of HCP (host cell protein) in antibody after antibody purification using ZZ-CSQ1 (Full) fusion protein.

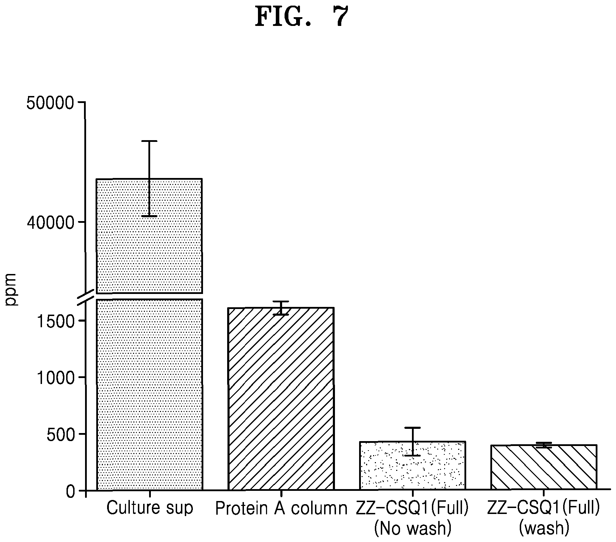

After antibody recovery by use of the ZZ-CSQ1 (Full) fusion protein according to Preparation Example 1, measurements were made to identify host cell protein (HCP). For HCP measurement in the purified antibody, CHO HCP ELILSA Kit (CANOPY) was used. 100 µl of the antibody sample eluted by using ZZ-CSQ1 (Full) was added to a 96-well plate to which anti-CHO antibodies were attached, and reacted at room temperature for 1.5 hours. After washing 3 times with 250 µl of 1X PBS-T, 100 µl of reporting antibodies were added to the plate, and the plate was covered with a lid and reacted at room temperature for 45 minutes. After washing 3 times with 250 µl of 1X PBS-T, 100 µl of Streptavidin-HRP (horseradish peroxidase) was added at a concentration of 0.1 µg/ml, and reacted at room temperature for 30 minutes. After washing 3 times with 250 µl of 1X PBS-T, tetramethylbenzidine (TMB) was treated and reacted for 10 minutes. Stop solution was added, and absorbance was measured at 450 nm by using a Microplate reader (BioTek). As a result, when using ZZ-CSQ1 (Full) according to Preparation Example 1, the HCP level was found reduced to 1/4 compared to Protein A Chromatography (FIG. 7).

### 1-6. Measurement of HCD (host cell DNA) in antibody after antibody purification using ZZ-CSQ1 (Full) fusion protein

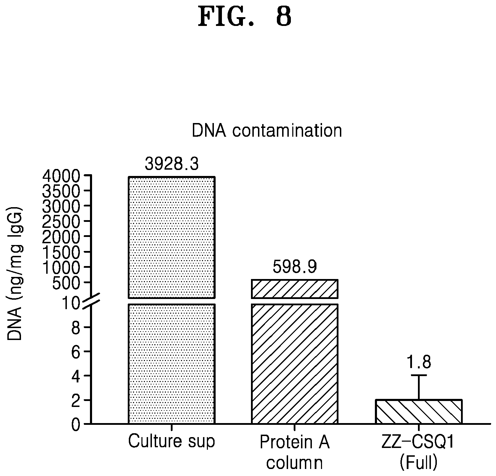

After antibody recovery by use of the ZZ-CSQ1 (Full) fusion protein according to Preparation Example 1, measurements were made to identify host cell DNA (HCD). For HCD measurement in the purified antibody, Quant-iT ds DNA High-Sensitivity Assay Kit (Invitrogen) was used. While protecting the working solution as provided from light, 200 µl of the working solution was put into a microplate and stored at room temperature for 3 hours. The purified antibody sample was added to each well and mixed, and fluorescence (excitation/emission maxima: 480/530 nm) was measured by using a microplate reader (PerkinElmer). The fluorescence was confirmed by repeatedly measuring three times. As a result, when using ZZ-CSQ1 (Full) according to Preparation Example 1, HCD level was found to be reduced to 1/332 compared to Protein A Chromatography (FIG. 8).

### 1-7. SEC-HPLC (size-exclusion high-performance liquid chromatography) analysis after antibody purification using ZZ-CSQ1 (Full) fusion protein.

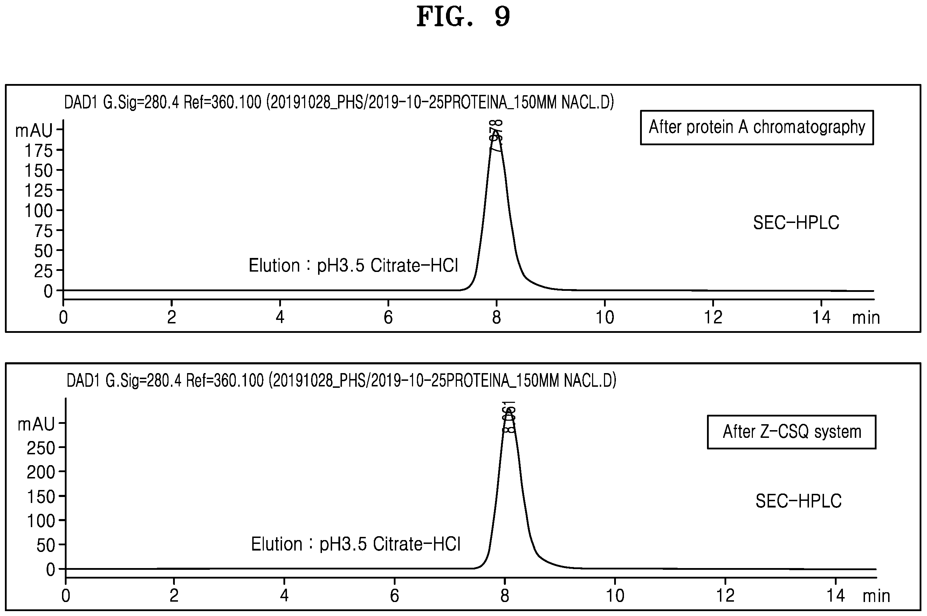

After antibody purification by use of the ZZ-CSQ1 (Full) fusion protein according to Preparation Example 1, SEC-HPLC analysis was conducted to examine aggregation of antibodies or ZZ-CSQ1 (Full). A Superdex 200 increase Size Exclusion Chromatography Column (SEC) was used. 50 mM citrate (pH 3.5) was used as an elution buffer, and 1 M Tris (pH 9.0) was immediately added for neutralization. 20 mM Tris (pH 7.5) + 150 mM NaCl was flowed at a flow rate of 0.2 ml/min for analysis, and the antibody sample purified by Protein A column was also analyzed under the same conditions as a control. As a result, the antibody quality obtained was found equivalent to that from Protein A Chromatography(FIG. 9).

### 1-8. Antibody recovery in elution of antibodies from Z6G-CSQ1 (Full) and ZL4G-CSQ1 (Full) fusion proteins

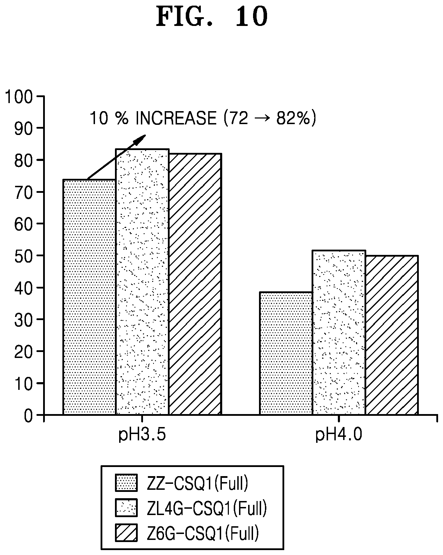

In order to increase antibody recovery yield, fusion proteins Z6G-CSQ1 (Full) according to Preparation Example 5 and ZL4G-CSQ1 (Full) according to Preparation Example 6 were made by replacing the ZZ domain in ZZ-CSQ1 (Full) according to Preparation Example 1 with Z6G (SEQ ID NO: 2) and ZL4G (SEQ ID NO: 3), respectively. To investigate the antibody recovery yieldby the fusion protein with a new domain, Z6G-CSQ1 (Full) and ZL4G-CSQ1 (Full) were added to the antibody culture medium at a 1:1 ratio, respectively, and incubated at 4 °C for 30 minutes. Then, 10 mM calcium was added and incubated at 4 °C for 30 minutes followed by centrifugation at 4,000 g for 30 minutes to separate the resulting medium into a supernatant and pellets. 50 mM citrate (pH 3.5) and 50 mM citrate (pH 4.0) buffer were added to the pellets and elution was conducted for 20 minutes. After elution, the eluate was separated intoa supernatant and pellets by centrifugation at 4,000 g for 30 minutes, 4X sample buffer was added to the supernatant, and the resulting mixture was heat treatmed at 80 °C for 10 minutes followed by SDS-PAGE (polyacrylamide gel electrophoresis) for identification of elution level of antibody. As a result, the antibody elution level was found increased by 10 % after changing the Z domain to Z6G and ZL4G compared to ZZCSQ1 (Full). In addition, after replacing the ZZ-domain with Z6G-CSQ1 (Full) according to Preparation Example 5 and ZL4G-CSQ1 (Full) according to Preparation Example 6, the recovery of the antibody was found higher than that of ZZCSQ1 (Full) according to Preparation Example 1 (FIG. 10).

### Experimental Example 2: Confirmation of reactivity and stability of fusion protein including calsequestrin consisting of amino acid sequence of SEQ ID NO: 6

### 2-1. Comparison of ZZ-CSQ2 (Full) and ZZ-CSQ2 (Δ)

### (1) Calcium reactivity

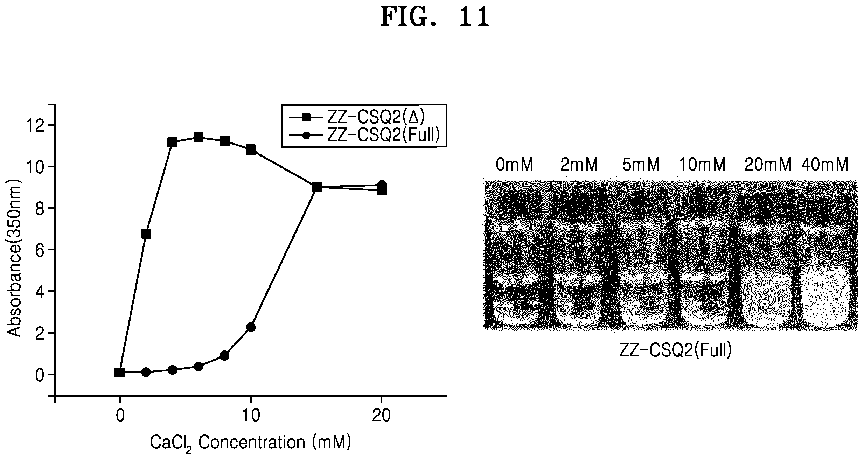

Turbidity and antibody elution by the ZZ-CSQ2 (full) fusion protein according to Preparation Example 7 and the ZZ-CSQ2 (Δ) fusion protein according to Comparative Example were measured in the same manner as in Experimental Example 1, and the results are shown in FIG. 11 .

### (2) Improvement of antibody recovery and purity

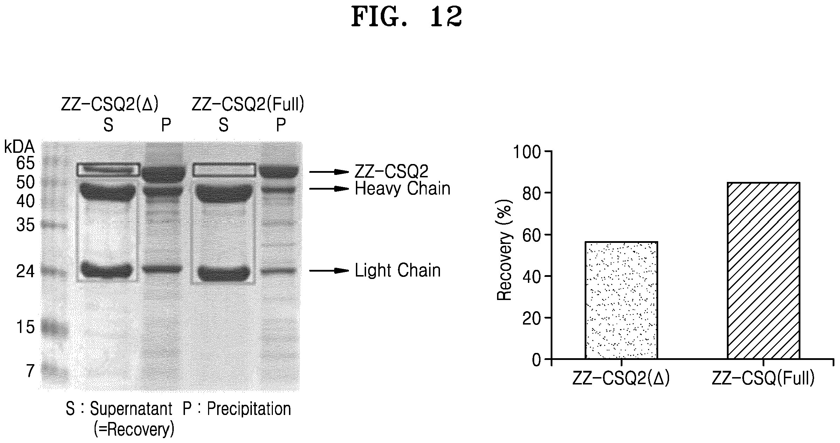

ZZ-CSQ2 (Δ) and ZZ-CSQ2 (Full) fusion proteins were respectively added to an antibody culture medium at a 1:1 ratio, and reacted at 4 °C for 30 minutes to examine the antibody elution recovery by the ZZ-CSQ2(Δ) and ZZ-CSQ2(Full) fusion proteins. After adding 20 mM CaCl₂ to the antibody+fusion protein mixture and reacting at 4 °C for 30 minutes, centrifugation was performed at 4,000 g for 30 minutes to separate the resulting mixture into a supernatant and pellets. 50 mM citrate (pH 3.5) buffer was added to the pellets for elution for 20 minutes. After elution, the supernatant and the pellets were separated by centrifugation at 4,000 g for 30 minutes. In order to identify elution level of antibodies from the fusion protein+antibody, 4X sample buffer was added to the eluate, the resulting mixture was heat-treated at 80 °C for 10 minutes followed by SDS-PAGE (polyacrylamide gel electrophoresis) for identification. As a result, it was found that about 60 % of the antibodies were recovered by using ZZ-CSQ2 (Δ), and more than 80 % of the antibodies were recovered by using ZZ-CSQ2 (Full). In addition, about 10 % of the antibodies recovered by using ZZ-CSQ2 (Δ) were found recovered together with the ZZ-CSQ2 (Δ) fusion proteins, but only less than 1 % of the antibodies recovered by using ZZ-CSQ2 (Full) were found recovered together with the ZZ-CSQ2 (Full) fusion proteins, thereby showing that high-purity antibodies may be recovered by use of ZZ-CSQ2 (Full) (FIG. 12).

### (3) Stability depending on type of linker

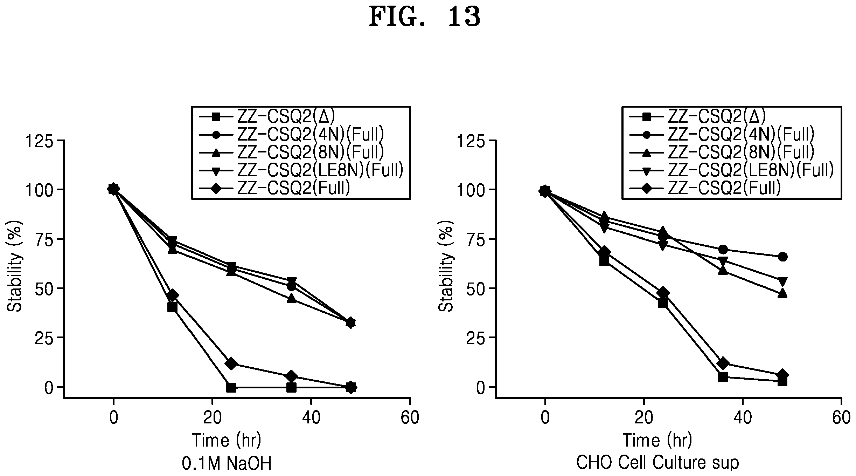

After changing G4S (SEQ ID NO: 7), which is a linker part of the ZZ-CSQ2 (Full) fusion protein, to each of 4N (SEQ ID NO: 8), 8N (SEQ ID NO: 9) and LE8N (SEQ ID NO: 10), a concentration of 1 mg/ml of each of the fusion proteins ZZ-CSQ2 (Full) and ZZ-CSQ2 (Δ) was treated with 0.1 M of NaOH and CHO Cell Culture Sup. in an incubator at 25 °C or 37 °C for 12, 24, 36, or 48 hours, in order to examine stability. In order to find any change in stability of the fusion protein over time, samples were taken at the indicated times, 4X sample buffer was added to each sample, then, the sample was heat-treated at 80 °C for 10 minutes followed by SDS-PAGE for identification. As a result, in case of ZZ-CSQ2 (Full) according to Preparation Examples 8 to 10, in which the linker was changed to 4N, 8N and LE8N in ZZ-CSQ2 (Full), respectively, stability was improved by 25 % when incubated in CHO Cell Culture sup for 24 hours, and stability was maintained 50 % or more for up to 36 hours in 0.1 M of NaOH, thereby confirming that the reuse rate may be increased (FIG. 13).

### 2-2. Purification of antibody by using ZZ-CSQ2 (Full) fusion protein

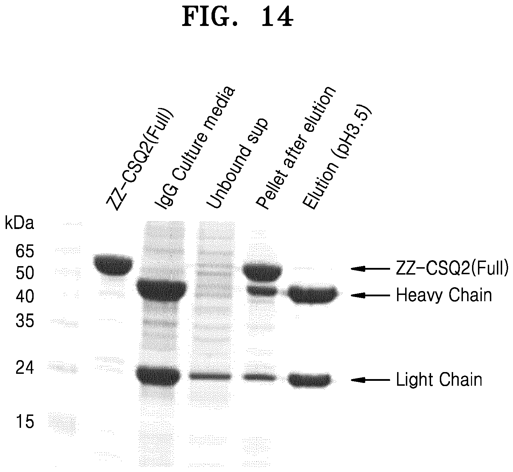

The process of purifying only antibodies from the antibody culture medium by using the ZZ-CSQ2 (Full) fusion protein was examined. ZZ-CSQ2 (Full) fusion protein was added to the antibody culture medium at a ratio of 1:1, and incubated at 4 °C for 30 minutes. 20 mM calcium was added to the resulting mixture and incubated at 4 °C for 30 minutes followed by centrifugation at 4,000 g for 30 minutes for separation thereof into a supernatant and pellets. 50 mM citrate (pH 3.5) was used to elute antibodies (IgG) from ZZ-CSQ2 (Full)+antibody (IgG). In order to confirm antibody recovery, 4X sample buffer was added to the eluate and the resulting mixture was heat-treated at 80 °C for 10 minutes followed by SDS-PAGE for identification. As a result, it was found that only antibodies (IgG) were eluted from the ZZ-CSQ2 (Full)+antibody (IgG) (FIG. 14).

### Experimental Example 3. Measurement of affinity of Z-CSQ1 (Full) fusion protein to antibody

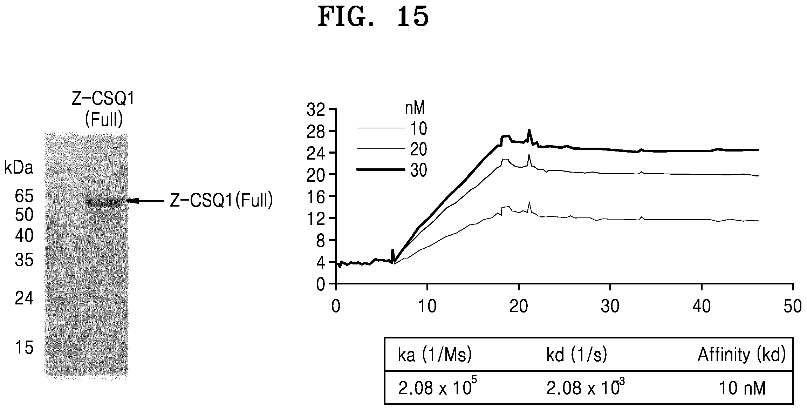

Z-CSQ1 (Full) was prepared by using a Z- domain consisting of SEQ ID NO: 13 instead of a ZZ-domain consisting of SEQ ID NO: 1 in Preparation Example 1, and SPR (BIACORE 3000) analysis was performed to measure affinity of the Z-CSQ1 (Full) fusion protein. Herceptin was immobilized on a CM5 chip, and Z-CSQ1 (Full) in PBS busffer at various concentrations (30, 60, 90 nM) was injected at a flow rate of 30 µl/min. Kinetics data were analyzed with BIA evaluation 2.1 software (GE Healthcare). As a result, the Z-domain of Z-CSQ1 was also found to be capable of binding strongly to the antibody (FIG. 15).

### Experimental Example 4. Measurement of affinity of (Z)5-CSQ1 (Full) fusion protein to antibody

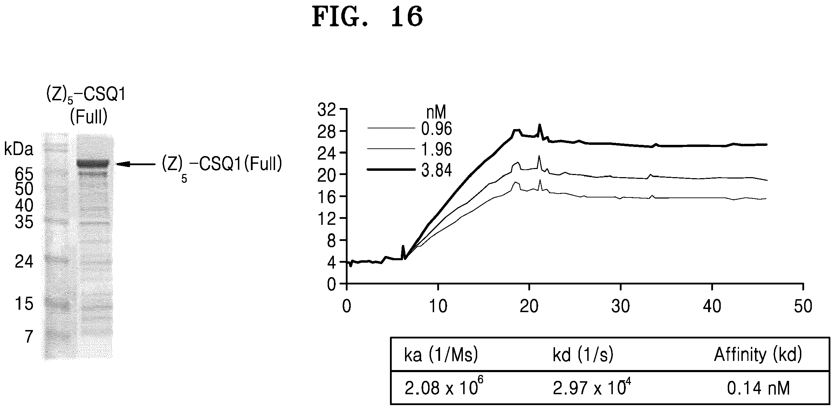

SPR (BIACORE 3000) analysis was performed to measure the affinity of (Z)₅-CSQ (Full) with five Z-domains. Herceptin was immobilized on a CM5 chip, and (Z)₅-CSQ (Full) in PBS buffer at various concentrations (0.96, 1.96, 3.84 nM) was injected at a flow rate of 30 µl/min. Kinetics data were analyzed with BIA evaluation 2.1 software (GE Healthcare). As a result, the Z-domain of (Z)₅-CSQ (Full) was also found to be capable of binding strongly to an antibody with affinity of 1.14 nM (FIG. 16).

### Experimental Example 5: Investigation of binding ratio of (Z)₅-CSQ1 (Full) fusion protein to antibodies

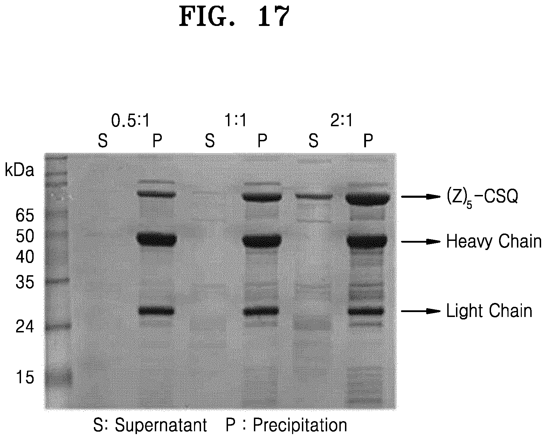

(Z)₅-CSQ1 (Full) fusion protein and antibodies were added at a ratio of 0.5:1, 1:1, and 2:1, and reacted at 4 °C for 30 minutes for binding. Calcium was added to the resulting reaction mixture of antibodies and the fusion protein, and incubated for reaction followed by centrifugation at 4,000 g at 4 °C for 30 minutes. After separating the supernatant and the pellets, the pellets were dissolved in 20 mM Tris-HCl (pH 7.0) buffer, and in order to identify the binding ratio of the fusion protein and antibodies, 4X sample buffer was added to the solution, and the resulting mixture was heat-treated at 80 °C for 10 minutes followed by SDS-PAGE for identfication. As a result, it was found that (Z)₅-CSQ1(Full) was completely precipitated with the antibodies even at ratios of 2:1 and 4:1(FIG. 17).

So far, the disclosure has been described with relevant embodiments. Those of ordinary skill in the art to which the present disclosure pertains will understand that the present disclosure may be implemented in modified forms without departing from the essential characteristics of the present disclosure. Therefore, the disclosed embodiments are to be considered in an illustrative rather than a limiting sense. The scope of the present disclosure is indicated in the claims rather than the foregoing description, and all differences within the scope equivalent thereto should be construed as being included in the present disclosure.

## Claims

1. A nucleic acid encoding a fusion protein of Z-domain and calsequestrin (Z-calsequestrin fusion protein) with improved reactivity, stability, and antibody recovery, wherein the calsequestrin consists of an amino acid sequence of SEQ ID NO: 5 or SEQ ID NO: 6.

2. The nucleic acid of claim 1, wherein the Z-domain is in a form of Zn, wherein the Z-domain is a repeating unit and n is an integer equal to or higher than 1.

3. The nucleic acid of claim 2, wherein the Z-domain consists of an amino acid sequence of SEQ ID NO: 13 and is in a form having 1 to 5 repeating units.

4. The nucleic acid of claim 1, wherein the Z-domain is a ZZ-domain consisting of an amino acid of SEQ ID NO: 1, a Z6G domain consisting of an amino acid of SEQ ID NO: 2, or a ZL4G domain consisting of an amino acid of SEQ ID NO: 3.

5. The nucleic acid of claim 1, wherein the Z-domain and the calsequestrin are fused via a linker consisting of one or more amino acid sequences selected from the group consisting of SEQ ID NOs: 7 to 10.

6. A recombinant expression vector including the nucleic acid according to any one of claims 1 to 5.

7. A host cell transformed with the recombinant expression vector according to claim 6.

8. A method of isolating and purifying antibodies with improved reactivity, stability, and antibody recovery, the method comprising:
A) preparing a recombinant vector including a nucleic acid encoding a Z-domain-calsequestrin fusion protein;
B) transferring the recombinant vector into a host cell to obtain a transformant;
C) expressing the Z-calsequestrin fusion protein in the transformant; and
D) isolating antibodies bount to Z-calsequestrin fusion protein from the expressed transformant by using calcium, wherein the calsequestrin consists of an amino acid sequence of SEQ ID NO: 5 or SEQ ID NO: 6.

9. The method of isolating and purifying antibodies with improved reactivity, stability, and antibody recovery according to claim 7, wherein the Z-domain is in a form of Zn, wherein the Z-domain is a repeating unit and n is an integer equal to or higher than 1.

10. The method of isolating and purifying antibodies with improved reactivity, stability, and antibody recovery according to claim 8, wherein the Z-domain is a ZZ-domain consisting of an amino acid of SEQ ID NO: 1, a Z6G domain consisting of an amino acid of SEQ ID NO: 2, or a ZL4G domain consisting of an amino acid of SEQ ID NO: 3.

11. The method of isolating and purifying antibodies with improved reactivity, stability, and antibody recovery according to claim 8, wherein the Z-domain and the calsequestrin are fused via a linker consisting of one or more amino acid sequences selected from the group consisting of SEQ ID NOs: 7 to 10.

12. The method of isolating and purifying antibodies with improved reactivity, stability, and antibody recovery according to claim 8, wherein step (D) is conducted under the conditions of pH 3.0 to pH 4.5.

13. The method of isolating and purifying antibodies with improved reactivity, stability, and antibody recovery according to claim 8, wherein step (D) of isolating antibodies bound to the Z-calsequestrin fusion protein from the expressed transformant by using calcium comprises:
(d-1) precipitating the Z-calsequestrin fusion protein-bound antibodies by using calcium; and
(d-2) removing the precipitate through centrifugation or filtration.
